# EUROPÄISCHE PATENTANMELDUNG

(11) **EP 4 043 445 A1**
(43) Veröffentlichungstag der Anmeldung: **17.08.2022**
(21) Anmeldenummer: 21156724.3
(22) Anmeldetag: 12.02.2021
(51) Int. Cl.: C07D 265/24, C07F 9/6571, C07F 9/6574

(54) **VERFAHREN ZUR HERSTELLUNG VON 2-AMINOBENZOXAZINONEN AUS 1-AZIDO-2-IODOBENZOLEN UND AMINEN**

(71) Anmelder: Evonik Operations GmbH, 45128 Essen (DE)
(72) Erfinder: WU, Xiao-Feng, 18059 Rostock (DE); ZHANG, Youcan, Shanghai (CN); BÖRNER, Armin, 18059 Rostock (DE); FRANKE, Robert, 45772 Marl (DE)
(74) Vertreter: Evonik Patent Association

(57) **Zusammenfassung**

Verfahren zur Herstellung von 2-Aminobenzoxazinonen aus 1-Azido-2-iodobenzolen und Aminen.

## Beschreibung

Die Erfindung betrifft ein Verfahren zur Herstellung von 2-Aminobenzoxazinonen aus 1-Azido-2-iodobenzolen und Aminen.

Die technische Aufgabe, welche der vorliegenden Erfindung zugrunde lag, ist die Bereitstellung eines Verfahrens, mit welchen 2-Aminobenzoxazinonen hergestellt werden können.

Diese Aufgabe wird gelöst durch ein Verfahren nach Anspruch 1.

Verfahren umfassend die Verfahrensschritte:
a) Vorlegen eines 1-Azido-2-iodobenzols;
b) Zugabe eines Liganden gemäß Formel (I): 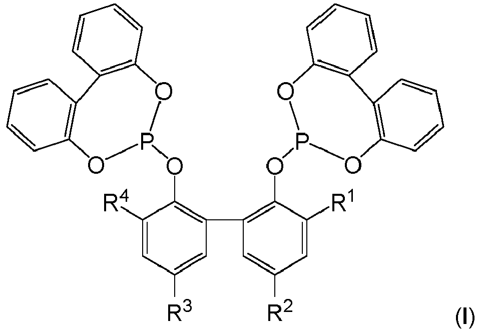 wobei
   R¹, R², R³, R⁴ jeweils unabhängig voneinander ausgewählt sind aus: -H, -(C₁-C₁₂)-Alkyl, -O-(C₁-C₁₂)-Alkyl;
c) Zugabe einer Verbindung, welche Pd umfasst;
d) Zugabe eines Amins;
f) Zuführen von CO;
g) Erwärmen des Reaktionsgemisches aus a) bis f), wobei das Alkylhalogenid zu einem Ester umgesetzt wird.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis d) vorgelegt wurden. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Der Begriff -(C₁-C₁₂)-Alkyl bzw. -O-(C₁-C₁₂)-Alkyl umfasst geradkettige und verzweigte Alkylgruppen mit 1 bis 12 Kohlenstoffatomen. Vorzugsweise handelt es sich dabei um -(C₁-C₈)-Alkylgruppen bzw. -O-(C₁-C₈)-Alkylgruppen, besonders bevorzugt um -(C₁-C₄)-Alkylgruppen bzw. -O-(C₁-C₄)-Alkylgruppen.

In einer Variante des Verfahrens stehen R¹ und R⁴ für -(C₁-C₁₂)-Alkyl.

In einer Variante des Verfahrens stehen R¹ und R⁴ für -*^{ter}*Bu.

In einer Variante des Verfahrens stehen R² und R³ für -O-(C₁-C₁₂)-Alkyl.

In einer Variante des Verfahrens stehen R² und R³ für -OCH₃.

In einer Variante des Verfahrens weist der Ligand die Struktur (1) auf:

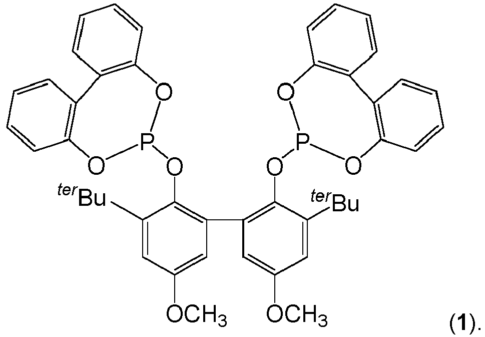

In einer Variante des Verfahrens weist das 1-Azido-2-iodobenzol die Formel **(II)** auf: 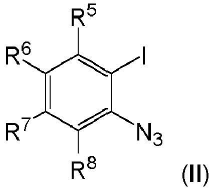 wobei R⁵, R⁶, R⁷, R⁸ jeweils unabhängig voneinander ausgewählt sind aus: -H, -(C₁-C₁₂)-Alkyl, -O-(C₁-C₁₂)-Alkyl.

In einer Variante des Verfahrens stehen R⁵, R⁶, R⁷, R⁸ für -H.

In einer Variante des Verfahrens ist die Verbindung, welche Pd umfasst, ausgewählt aus: PdCl₂, PdBr₂, Pd(OAc)₂, Pd/C, Pd(dba)₂ (dba = dibenzylidene acetone), PdCl₂(CH₃CN)₂.

In einer Variante des Verfahrens erfolgt das Zuführen von CO in Verfahrensschritt f) mit einem Druck in dem Bereich von 0,2 bis 3 MPa (2 bis 30 bar).

In einer Variante des Verfahrens erfolgt das Erwärmen des Reaktionsgemisches in Verfahrensschritt g) auf eine Temperatur in dem Bereich von 50 °C bis 120 °C.

In einer Variante des Verfahrens umfasst das Verfahren den zusätzlichen Verfahrensschritt f'): f') Zugabe einer Base.

In einer Variante des Verfahrens ist die Base ausgewählt aus: NEt₃, DiPEA, Pyridin, K₂OP₃, NaHCO₃, K₃PO₄.

In einer Variante des Verfahrens umfasst das Verfahren den zusätzlichen Verfahrensschritt f"):
f") Zugabe eines Lösungsmittels.

In einer Variante des Verfahrens ist das Lösungsmittel ausgewählt aus: CH₃CN, DMSO, DMF, *^{tert}*BuOH, THF, 1,4-Dioxan.

Im Folgenden soll die Erfindung anhand eines Ausführungsbeispiels näher erläutert werden.

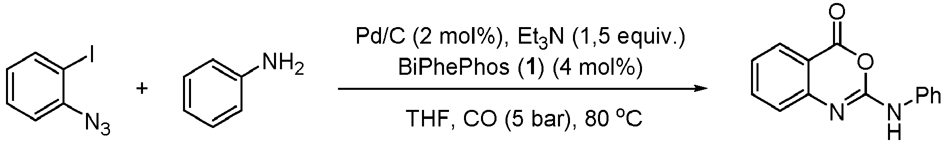

BiPhePhos **(1):**

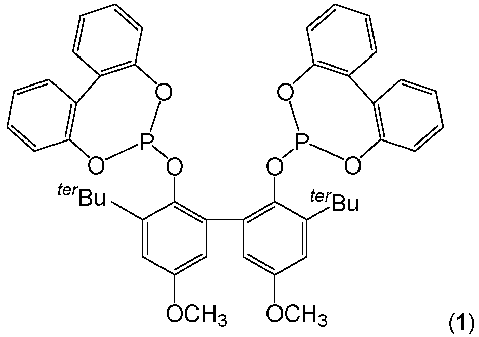

In eine Schraubdeckelflasche (4 mL), die mit einem Septum, einer kleinen Kanüle und einem Rührstab ausgestattet war, wurde 1-Azido-2-iodobenzol (0,3 mmol), Pd/C (2,0 mol%) und BiPhePhos (4,0 mol%) zugegeben. Die Fläschchen wurden dann dreimal mit Argon gespült, bevor Et₃N (64 mL, 1,5 Äquiv.) und getrocknetes THF (2 mL) zugegeben wurden. Diese Fläschchen wurden auf eine Alloyplatte gestellt und unter Luft in einen 300-ml-Autoklaven der Serie 4560 von Parr Instruments überführt. Nach dreimaligem Spülen des Autoklaven mit CO wurde ein Druck von 5 bar CO eingestellt und die Reaktion für 20 Stunden bei 80 °C durchgeführt. Danach wurde der Autoklav auf Raumtemperatur abgekühlt und der Druck wurde vorsichtig entspannt. Das Lösungsmittel wurde unter vermindertem Druck entfernt und die Rohprodukte wurden durch Säulenchromatographie auf Kieselgel (Eluent: Pentan/Ethylacetat = 5:1) gereinigt.

Der Versuch wurde unter analogen Bedingungen mit den Vergleichsliganden PPh₃ (2), DPPP **(3)** (DPPP = 1,3-Bis(diphenylphosphino)propan) und DPPF **(4)** (DPPF = 1,1'-Bis(diphenylphosphino)ferrocen) widerholt.

Reaktionsbedingungen:
T = 80 °C, p(CO) = 5 bar, t = 20 h, Lösungsmittel = THF.

Die Versuchsergebnisse sind in der nachfolgenden Tabelle aufgeführt:

| Ligand | Ausbeute [%] |
|---|---|
| **(1)*** | 35 |
| **(2)** | 0 |
| **(3)** | 0 |
| **(4)** | 0 |

| | |
|---|---|
| * erfindungsgemäßes Verfahren | |

Mit dem erfindungsgemäßen Verfahren konnte, im Gegensatz zu den Vergleichsversuchen, das gewünschte Produkt hergestellt werden.

Wie das Ausführungsbeispiel zeigt, wird die Aufgabe durch das erfindungsgemäße Verfahren gelöst.

## Patentansprüche

1. Verfahren umfassend die Verfahrensschritte:
a) Vorlegen eines 1-Azido-2-iodobenzols;
b) Zugabe eines Liganden gemäß Formel (**I**): 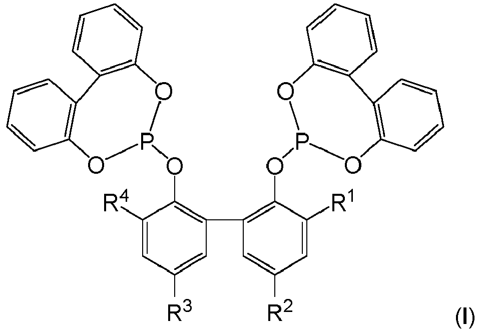 wobei
R¹, R², R³, R⁴ jeweils unabhängig voneinander ausgewählt sind aus: -H, -(C₁-C₁₂)-Alkyl, -O-(C₁-C₁₂)-Alkyl;
c) Zugabe einer Verbindung, welche Pd umfasst;
d) Zugabe eines Amins;
f) Zuführen von CO;
g) Erwärmen des Reaktionsgemisches aus a) bis f), wobei das Alkylhalogenid zu einem Ester umgesetzt wird.

2. Verfahren nach Anspruch 1,
wobei R¹ und R⁴ für -(C₁-C₁₂)-Alkyl stehen.

3. Verfahren nach einem der Ansprüche 1 oder 2,
wobei R¹ und R⁴ für -*^{ter}*Bu stehen.

4. Verfahren nach einem der Ansprüche 1 bis 3,
wobei R² und R³ für -O-(C₁-C₁₂)-Alkyl stehen.

5. Verfahren nach einem der Ansprüche 1 bis 4,
wobei R² und R³ für -OCH₃ stehen.

6. Verfahren nach einem der Ansprüche 1 bis 5,
wobei der Ligand die Struktur (1) aufweist:

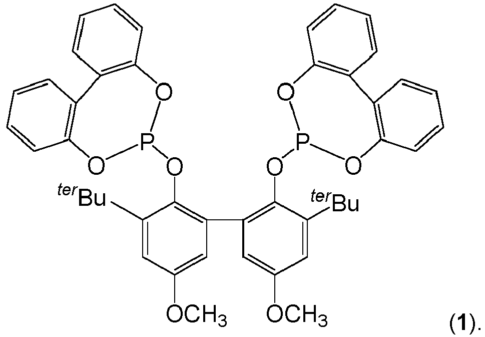

7. Verfahren nach einem der Ansprüche 1 bis 6,
wobei das 1-Azido-2-iodobenzol die Formel (**II**) aufweist: 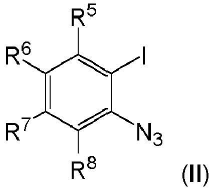 wobei R⁵, R⁶, R⁷, R⁸ jeweils unabhängig voneinander ausgewählt sind aus: -H, -(C₁-C₁₂)-Alkyl, -O-(C₁-C₁₂)-Alkyl.

8. Verfahren nach Anspruch 7,
wobei R⁵, R⁶, R⁷, R⁸ für -H stehen.

9. Verfahren nach einem der Ansprüche 1 bis 8,
wobei die Verbindung, welche Pd umfasst, ausgewählt ist aus: PdCl₂, PdBr₂, Pd(OAc)₂, Pd/C, Pd(dba)₂ (dba = dibenzylidene acetone), PdCl₂(CH₃CN)₂.

10. Verfahren nach einem der Ansprüche 1 bis 9,
wobei das Zuführen von CO in Verfahrensschritt f) mit einem Druck in dem Bereich von 0,2 bis 3 MPa (2 bis 30 bar) erfolgt.

11. Verfahren nach einem der Ansprüche 1 bis 10,
wobei das Erwärmen des Reaktionsgemisches in Verfahrensschritt g) auf eine Temperatur in dem Bereich von 50 °C bis 120 °C erfolgt.

12. Verfahren nach einem der Ansprüche 1 bis 11,
umfassend den zusätzlichen Verfahrensschritt f'):
f') Zugabe einer Base.

13. Verfahren nach Anspruch 12,
wobei die Base ausgewählt ist aus: NEt₃, DiPEA, Pyridin, K₂CO₃, NaHCO₃, K₃PO₄.

14. Verfahren nach einem der Ansprüche 1 bis 13,
umfassend den zusätzlichen Verfahrensschritt f"):
f") Zugabe eines Lösungsmittels.

15. Verfahren nach Anspruch 14,
wobei das Lösungsmittel ausgewählt ist aus: CH₃CN, DMSO, DMF, *^{terf}*BuOH, THF, 1,4-Dioxan.
